(19) **Europäisches Patentamt / European Patent Office / Office européen des brevets**

(11) **EP 4 678 285 A1**

(12) **EUROPEAN PATENT APPLICATION**
published in accordance with Art. 153(4) EPC

(43) Date of publication:
**14.01.2026 Bulletin 2026/03**

(21) Application number: **24767473.2**

(22) Date of filing: **15.02.2024**

(51) International Patent Classification (IPC):
*B01J 23/72* (2006.01)    *B01J 21/08* (2006.01)
*B01J 35/51* (2024.01)    *B01J 35/40* (2024.01)
*B01J 37/03* (2006.01)    *B01J 37/02* (2006.01)
*C07C 29/141* (2006.01)    *C07C 31/20* (2006.01)

(52) Cooperative Patent Classification (CPC):
(C-Sets available)
**C07C 29/141; B01J 21/08; B01J 23/72;**
**B01J 35/51; B01J 35/58; B01J 37/0221;**
**B01J 37/03**                    (Cont.)

(86) International application number:
**PCT/KR2024/095165**

(87) International publication number:
**WO 2024/186187 (12.09.2024 Gazette 2024/37)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority:  **03.03.2023 KR 20230028645**
                **14.02.2024 KR 20240020773**

(71) Applicant: **LG Chem, Ltd.**
**Yeongdeungpo-gu**
**Seoul 07336 (KR)**

(72) Inventors:
 • **SUH, Myungji**
   **Daejeon 34122 (KR)**

 • **JUNG, Euiyoung**
   **Daejeon 34122 (KR)**
 • **KIM, Tae Yun**
   **Daejeon 34122 (KR)**
 • **YANG, Sungpil**
   **Daejeon 34122 (KR)**
 • **YANG, Jusang**
   **Daejeon 34122 (KR)**
 • **EOM, Sungshik**
   **Daejeon 34122 (KR)**

(74) Representative: **Goddar, Heinz J.**
**Boehmert & Boehmert**
**Anwaltspartnerschaft mbB**
**Pettenkoferstrasse 22**
**80336 München (DE)**

(54) **CATALYST FOR PREPARING NEOPENTYL GLYCOL AND PREPARATION METHOD THEREFOR**

(57)    The present invention provides a catalyst for preparing neopentyl glycol and a method for preparing a catalyst for preparing neopentyl glycol. The catalyst for preparing neopentyl glycol according to the present invention is characterized by improving the strength, reactivity and stability of the catalyst by controlling the weight ratio of Cu and Si and the content of CuO measured by XRD analysis. Since the catalyst for preparing neopentyl glycol of the present invention may improve the strength of the catalyst and may maintain the activity of the catalyst even in a high-temperature and high-pressure reaction, it is possible to increase the stability of the process, and the yield of neopentyl glycol is high.

[Figure 1]

**(Cont. next page)**

(52) Cooperative Patent Classification (CPC): (Cont.)

C-Sets
**C07C 29/141, C07C 31/20**

**Description**

[Technical Field]

The present invention relates to a catalyst for preparing neopentyl glycol and a method for preparing the same.
This application claims priority to and the benefit of Korean Patent Application Nos. 10-2023-0028645 and 10-2024-0020773 filed in the Korean Intellectual Property Office on March 3, 2023 and February 14, 2024, respectively, the entire contents of which are incorporated herein by reference.

[Background Art]

Neopentyl glycol (NPG) is a white crystalline material with a melting point of 130°C or higher and is used as an important intermediate for various synthetic resins, and is also widely used industrially as a raw material for various plastic powder coatings, synthetic lubricants, plasticizers, surfactants, fiber processing agents, and the like.
Such NPG is generally prepared by subjecting isobutyraldehyde and formaldehyde to an aldol condensation reaction to prepare hydroxypivaldehyde (HPA), and then reacting this HPA with hydrogen in the presence of a catalyst. In this case, in order to obtain a high yield, the reaction is carried out in the presence of a copper-based catalyst under high temperature (130°C or higher) and high pressure (35 bar or higher) conditions.
Since the reaction is carried out under such high temperature and high pressure conditions, a problem in that the strength of the catalyst decreases may occur. When the strength of the catalyst decreases, this may cause problems in terms of process stability.
In addition, since the reaction is performed under high temperature and high pressure conditions, catalyst support components are eluted, so that the catalyst may be physically crushed, increasing the differential pressure inside a reactor and making it impossible to operate the production process.
Therefore, continuous efforts are being made to develop a catalyst with improved catalyst stability and excellent catalyst activity due to excellent catalyst strength even under high temperature and high pressure conditions. In addition, continuous efforts are being made to develop technologies capable of preventing the elution of the catalyst support.
[Citation List] (Patent Document 1) Korean Patent Application Laid-Open No. 10-2011-0038324

[Detailed Description of the Invention]

[Technical Problem]

The present invention has been made in an effort to provide a catalyst for preparing neopentyl glycol having excellent catalyst strength and catalyst activity, and a method for preparing the same.

[Technical Solution]

An exemplary embodiment of the present invention provides a catalyst for preparing neopentyl glycol (NPG), including: a support; a fiber-based binder; and CuO and $SiO_2$, in which the weight ratio of Cu and Si is 30 : 70 to 70 : 30, and the catalyst for preparing neopentyl glycol includes 5 parts by weight to 40 parts by weight of CuO based on 100 parts by weight of the catalyst for preparing neopentyl glycol by XRD analysis.
An exemplary embodiment of the present invention provides a method for preparing a catalyst for preparing neopentyl glycol, the method including: preparing a coprecipitate including a copper (Cu) precursor and a silica ($SiO_2$) sol; drying the coprecipitate; preparing a coating powder by adding a fiber-based binder to the dried coprecipitate; and coating a support with the coating powder using an aqueous alcohol solution, in which the catalyst for preparing neopentyl glycol is the above-described catalyst for preparing neopentyl glycol.

[Advantageous Effects]

Since the catalyst for preparing neopentyl glycol according to the present invention can maintain the activity of the catalyst even in a high-temperature and high-pressure reaction due to the excellent strength, reactivity and stability of the catalyst, the stability of the process for preparing neopentyl glycol can be enhanced. That is, since the process can be carried out for a long time, productivity can be increased.
Since the catalyst for preparing neopentyl glycol according to the present invention has excellent catalyst activity, the yield of neopentyl glycol is high when neopentyl glycol is prepared using the catalyst.

[Brief Description of Drawings]

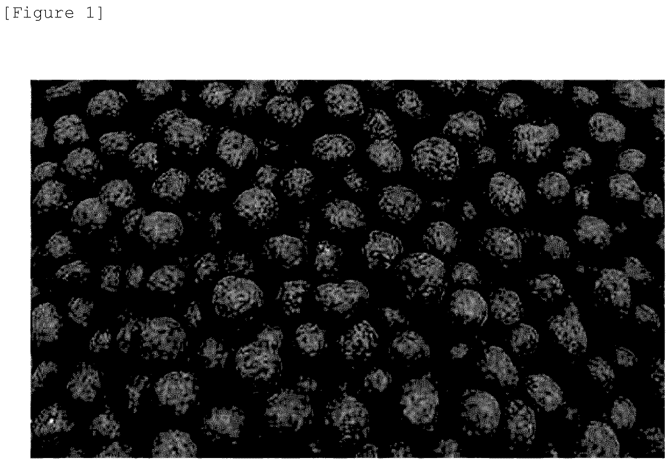

FIG. 1 is a photographed image illustrating the catalyst of Example 1.

[Best Mode]

Hereinafter, the present invention will be described in detail such that a person skilled in the art to which the present invention pertains can easily carry out the present invention. However, the present invention may be implemented in various different forms, and is not limited to the configurations described herein.

When one part "includes" one constituent element in the present specification, unless otherwise specifically described, this does not mean that another constituent element is excluded, but means that another constituent element may be further included.

In the present specification, 'p to q' or 'p~q' means 'p or more and q or less'.

In the present specification, "Dn" means the particle diameter distribution, and means the particle diameter at the n% point of the cumulative distribution of the number of particles according to the particle diameter. That is, D50 is the particle diameter (average particle diameter) at the 50% point of the cumulative distribution of the number of particles according to the particle diameter, D90 is the particle diameter at the 90% point of the cumulative distribution of the number of particles according to the particle diameter, and D10 is the particle diameter at the 10% point of the cumulative distribution of the number of particles according to the particle diameter. Meanwhile, the particle diameter distribution may be measured using a laser diffraction method. Specifically, after a powder to be measured is dispersed in a dispersion medium, a particle size distribution is calculated by introducing the resulting dispersion into a commercially available laser diffraction particle size measurement device (for example, Microtrac S3500) to measure the difference in diffraction pattern according to the particle size when particles pass through the laser beam.

In the present specification, the "fiber-based binder" means a binder including a fibrous material, and the specific types thereof will be described below.

Further, in describing the present invention, detailed descriptions of related known techniques that may unnecessarily obscure the gist of the present invention will be omitted.

An exemplary embodiment of the present invention provides a catalyst for preparing neopentyl glycol (NPG), including: a support; a fiber-based binder; and CuO and $SiO_2$, in which the weight ratio of Cu and Si is 30 : 70 to 70 : 30, and the catalyst for preparing neopentyl glycol includes 5 parts by weight to 40 parts by weight of CuO based on 100 parts by weight of the catalyst for preparing neopentyl glycol by XRD analysis.

That is, the catalyst for preparing neopentyl glycol according to the present invention is a copper-based catalyst for preparing neopentyl glycol.

In addition, the catalyst for preparing neopentyl glycol according to the present invention corresponds to a coated catalyst in which a support is coated with a catalyst component.

In order to obtain the weight ratio of Cu and Si, the content (wt%) of Cu may be measured by Inductively Coupled Plasma Optical Emission Spectrometer (ICP-OES) analysis. More specifically, the content may be measured using an Optima 8300DV apparatus manufactured by Perkin Elmer Inc. After the content of Cu is obtained using the following Mathematical Formula 1, the content of Si may be obtained using the following Mathematical Formula 2.

Content (wt%) of Cu = concentration to be measured ($\mu$g/mL) $\times$ (diluted volume (mL) / weight ($\mu$g) of catalyst) $\times$ 100 [Mathematical Formula 1]

[Mathematical Formula 2]

Content (wt%) of Si = 100 wt% - content (wt%) of Cu

The catalyst for preparing neopentyl glycol according to the present invention may enhance the strength, reactivity and stability of the catalyst by including a support and a fiber-based binder. In other words, the catalyst for preparing neopentyl glycol according to the present invention may maintain the activity of the catalyst even in a high-temperature and high-pressure reaction due to the excellent strength, reactivity and stability of the catalyst. Furthermore, since the catalyst for preparing neopentyl glycol according to the present invention has the weight ratio of Cu and Si and excellent activity of the catalyst by including CuO in the above content range, there is an advantage in that the yield of neopentyl glycol is high when neopentyl glycol is prepared using the catalyst.

In an exemplary embodiment of the present invention, the catalyst for preparing neopentyl glycol may include CuO in an amount of 5 parts by weight to 40 parts by weight, preferably 10 parts by weight to 35 parts by weight, more preferably 10 parts by weight to 30 parts by weight, and even more preferably 5 parts by weight to 20 parts by weight, based on 100 parts by weight of the catalyst for preparing neopentyl glycol, by XRD analysis.

As described above, the catalyst for preparing neopentyl glycol according to the present invention has even better catalyst activity when the weight ratio of Cu and Si and the content of CuO satisfy the above ranges, so that the yield of neopentyl may be increased when neopentyl glycol is prepared using the catalyst.

In an exemplary embodiment of the present invention, the catalyst for preparing neopentyl glycol may include $CuO/SiO_2$, $CuO/BaO/SiO_2$, $CuO/ZnO/SiO_2$, $CuO/Al_2O_3/SiO_2$, $CuO/MnO/SiO_2$ or $CuO/Cr_2O_3/SiO_2$, but is not limited thereto. That is, the catalyst for preparing neopentyl glycol according to the present invention is a copper-based catalyst for preparing neopentyl glycol.

In an exemplary embodiment of the present invention, the fiber-based binder may be one or more selected from the group consisting of glass fiber, carbon fiber, aramid fiber, alumina fiber, silicon carbide fiber, and boron fiber. When the fiber-based binder is included, there is an effect that the strength of the catalyst is easily increased by binding to the support and the reactivity is increased by increasing the degree of dispersion of copper.

In an exemplary embodiment of the present invention, the support may have a packing density of 600 $kg/m^3$ to 1500 $kg/m^3$.

In an exemplary embodiment of the present invention, the support may have a strength of 30 N to 250 N, preferably 40 N to 200 N.

In an exemplary embodiment of the present invention, the support may have a packing density of 600 $kg/m^3$ to 1,500 $kg/m^3$, preferably 700 $kg/m^3$ to 1,400 $kg/m^3$.

In an exemplary embodiment of the present invention, the support may include zirconium oxide, aluminum silicate oxide, silicon oxide or aluminum oxide.

When the support satisfies the above-described strength and packing density or includes the oxide type, there is an effect that both the catalyst reactivity and the catalyst stability described above are satisfied. In the present specification, the "strength" refers to the measurement of the desorption strength of a single catalyst, and is specifically calculated from the average value of 20 maximum desorption strengths at the initial collapse point using FGN-50B manufactured by SHIMPO. That is, in the present invention, the strength may mean a strength measured by the measurement method.

In the present specification, the "packing density" is a value calculated by measuring apparent density, and is specifically a value obtained by measuring the weight of an empty measuring cylinder, filling the measuring cylinder with 100 cc of a molded catalyst, measuring the weight, and dividing the difference in weight by the volume of the molded catalyst. That is, in the present invention, the packing density may mean an apparent density measured by the measurement method.

In an exemplary embodiment of the present invention, the shape of the support may be a sphere type, a cylinder type, a flat surface type, and the like, but is not limited thereto.

In an exemplary embodiment of the present invention, the support may be a sphere-type support, and the sphere-type support may have an average particle diameter of 3 mm to 7 mm.

In an exemplary embodiment of the present invention, the sphere-type support may have an average particle diameter of 3 mm to 7 mm, preferably 3 mm to 5 mm. The average particle diameter means the particle diameter (D50) at a point of 50% of the particle number cumulative distribution depending on the particle diameter, as described above.

When the sphere-type support satisfies the average particle diameter range, there is an effect of further improving the above-described catalyst reactivity and stability.

An exemplary embodiment of the present invention provides a method for preparing a catalyst for preparing neopentyl glycol, the method including: preparing a coprecipitate including a copper (Cu) precursor and a silica ($SiO_2$) sol; drying the coprecipitate; preparing a coating powder by adding a fiber-based binder to the dried coprecipitate; and coating a support with the coating powder using an aqueous alcohol solution, in which the catalyst for preparing neopentyl glycol is the above-described catalyst for preparing neopentyl glycol. For reference, silica corresponds to another expression for silicon dioxide.

In an exemplary embodiment of the present invention, the copper precursor may be $Cu(NO_3)_2 \cdot 3H_2O$, but is not limited thereto.

In an exemplary embodiment of the present invention, the coprecipitate may further include sodium hydroxide. The sodium hydroxide may serve as a precipitant to prepare a coprecipitate.

The method for preparing a catalyst for preparing neopentyl glycol according to an exemplary embodiment of the present invention may further include preparing an aqueous alcohol solution. The type and content of the aqueous alcohol solution described below may be used.

The method for preparing a catalyst for preparing neopentyl glycol according to an exemplary embodiment of the present invention may further include filtering and washing the coprecipitate before drying the coprecipitate. The washing may be performed with distilled water.

In an exemplary embodiment of the present invention, the fiber-based binder may be included in an amount of 5 parts by weight to 20 parts by weight, preferably 5 parts by weight to 15 parts by weight based on 100 parts by weight of the coprecipitate. The above-described content may be applied to the type of fiber-based binder, and when the fiber-based binder in the coprecipitate satisfies the above content, the fiber-based binder may more easily bind to the support and may enhance the activity of the catalyst.

In an exemplary embodiment of the present invention, the drying of the coprecipitate may be performed at a temperature of 80°C to 120°C for 12 to 48 hours, but is not limited thereto, and depending on the total amount of the coprecipitate, temperature and time conditions may be changed. A drying oven may be used for the drying.

In an exemplary embodiment of the present invention, the preparing of the coating powder by adding the fiber-based binder to the dried coprecipitate may include: pulverizing a material in which the fiber-based binder is added to the dried coprecipitate; and preparing a powder by classifying the pulverized material.

In an exemplary embodiment of the present invention, the classification range of the powder may be 5 $\mu$m to 100 $\mu$m, preferably 5 $\mu$m to 85 $\mu$m. In the present specification, classification means dividing a powder/grain having the same density into two or more particle groups depending on the particle diameter. That is, the classification range of the powder means that the particle diameter of the powder is 5 $\mu$m to 100 $\mu$m, preferably 5 $\mu$m to 85 $\mu$m, and the powder may be classified depending on the diameter. When the above classification range is satisfied, it is easier to enhance the activation of the catalyst.

In an exemplary embodiment of the present invention, the coating of the support with the coating powder using an aqueous alcohol solution may include introducing the support into a sugar coating machine; and spraying the coating powder and the aqueous alcohol solution onto the support while rotating the sugar coating machine, but is not limited thereto, and any method may be used as long as it can coat the support with the coating powder and the aqueous alcohol solution.

In an exemplary embodiment of the present invention, the aqueous alcohol solution may be an aqueous solution including one or more selected from the group consisting of methanol, ethanol, and isopropyl alcohol.

In an exemplary embodiment of the present invention, the concentration of the aqueous alcohol solution may be 0.1 parts by weight to 80 parts by weight of alcohol based on 100 parts by weight of the alcohol aqueous solution.

The method for preparing a catalyst for preparing neopentyl glycol according to an exemplary embodiment of the present invention may further include: drying the support coated with the coating powder; and firing the dried support coated with the coating powder.

In an exemplary embodiment of the present invention, the drying of the support coated with the coating powder may be performed at a temperature of 80°C to 120°C for 6 hours to 24 hours, but is not limited thereto, and the temperature and time conditions may be changed depending on the total amount of the coating powder and the aqueous alcohol solution and the size of the support. However, the support may be dried for a time shorter than the time for which the coprecipitate is dried. Even in this case, a drying oven may be used for the drying.

In an exemplary embodiment of the present invention, the firing of the dried support coated with the coating powder may be performed at a temperature of 250°C to 650°C for 2 hours to 8 hours. A firing furnace may be used for the firing.

In an exemplary embodiment of the present invention, the firing of the dried support coated with the coating powder may be performed at a temperature of 250°C to 650°C, preferably 250°C to 450°C.

In an exemplary embodiment of the present invention, the firing of the dried support coated with the coating powder may be performed for 2 hours to 8 hours, preferably 3 hours to 5 hours.

In an exemplary embodiment of the present invention, the firing of the dried support coated with the coating powder may be performed in an air atmosphere.

An exemplary embodiment of the present invention provides a method for preparing neopentyl glycol, the method including carrying out a hydrogenation reaction by introducing a hydroxypivaldehyde (HPA) solution and hydrogen into a hydrogenation reactor, in which the hydrogenation reactor includes the catalyst for preparing neopentyl glycol according to the present invention.

In an exemplary embodiment of the present invention, the method for preparing neopentyl glycol may be a method known in the art, except for including the catalyst for preparing neopentyl glycol according to the present invention.

For example, the hydrogenation reactor may be a fixed bed reactor (FBR) packed with a catalyst for preparing neopentyl glycol, and in this case, the catalyst and the reaction product need not be separated, and the hydrogenation reactor is stably operated and economical because it is possible to lower the reaction temperature and reaction pressure compared to the related art, and has an effect of significantly reducing investment costs because the catalyst is easily replaced and the size of the reactor may be reduced.

In an exemplary embodiment of the present invention, the hydroxypivaldehyde solution may include 50 wt% to 80 wt% of hydroxypivaldehyde, 1 wt% to 5 wt% of neopentyl glycol, 15 wt% to 35 wt% of alcohol, 1 wt% to 10 wt% of water, and 1 wt% to 10 wt% of hydroxypivalyl hydroxypivalate (hereinafter, HPNE), and in this case, there is an effect of suppressing the production of by-products because the heat of reaction may be minimized without reducing the reactivity.

Furthermore, in an exemplary embodiment of the present invention, the hydrogenation reaction may be performed at a reaction temperature of 100°C to 250°C, preferably 100°C to 200°C, and more preferably 100°C to 180°C.

Further, in an exemplary embodiment of the present invention, the hydrogenation reaction may be performed at a reaction pressure of 35 bar or more. The reaction pressure means the measured pressure.

When the catalyst for preparing neopentyl glycol according to an exemplary embodiment of the present invention is applied to the preparation of neopentyl glycol, it is possible to prevent a problem in that catalyst components (for example, copper components) in a neopentyl glycol solution to be prepared are eluted.

Therefore, when the catalyst for preparing neopentyl glycol according to an exemplary embodiment of the present invention is applied to the preparation of neopentyl glycol, there is no need for a purification process due to the elution of catalyst components (for example, copper components), and the catalyst life is extended, so that it is possible to obtain an effect capable of lowering preparation costs.

[Mode for Invention]

Hereinafter, the present invention will be described in detail with reference to Examples for specifically describing the present invention. However, the Examples according to the present invention may be modified in various forms, and it is not interpreted that the scope of the present invention is limited to the Examples to be described in detail below. The Examples of the present invention are provided for more completely explaining the present invention to the person with ordinary skill in the art.

**<Example 1>**

A coprecipitate in the form of a slurry was prepared by introducing a silica sol and NaOH into an aqueous solution of $Cu(NO_3)_2 \cdot 3H_2O$ for coprecipitation, such that the weight ratio of Cu and Si satisfied Cu:Si=40:60. The coprecipitate in the form of a slurry was filtered and washed with distilled water to obtain a coprecipitate in the form of a cake. The coprecipitate cake was dried in an oven at a temperature of 80°C for 15 hours.

Subsequently, 7 wt % of ceramic wool corresponding to a fiber-based binder was added to the dried coprecipitate cake based on the total weight of the dried coprecipitate cake. After the ceramic wool was added, the coprecipitate cake to which the ceramic wool was added was pulverized to prepare a mixed powder (coating powder) classified to 45 $\mu$m or less. Thereafter, in order to coat a support to be described below with the mixed powder, the mixed powder, isopropyl alcohol (IPA), and water were mixed to prepare a mixed solution. Here, ceramic wool means a type of fiber-based binder which is formed in the form of a roll by stacking ceramic fibers and sewing and punching the layers.

Thereafter, an alumina ball with a size of 3.2 mm (strength of about 150 N) corresponding to the support was put into a chamber, and the alumina ball was coated with the mixed solution while spraying the mixed solution. In this case, the coating thickness was 2 mm to 4 mm. For reference, the coating thickness means a calculated value obtained by dividing a value obtained by subtracting the size of the alumina ball from the size of the catalyst for preparing neopentyl glycol by 2.

After the alumina ball was completely coated, the alumina ball coated with the mixed solution was dried at 80°C for 7 hours using an oven, and the dried alumina ball coated with the mixed solution was fired in a firing furnace at a temperature of 250°C to 550°C for 8 hours to prepare the catalyst for preparing neopentyl glycol in Example 1.

FIG. 1 is a photographed image illustrating the catalyst of Example 1 prepared by the method.

**<Examples 2 to 9 and Comparative Examples 1 to 4, 7, and 8>**

Catalysts for preparing neopentyl glycol in Examples 2 to 9 and Comparative Examples 1 to 4, 7, and 8 were prepared in the same manner as in Example 1, except that in Example 1, a coprecipitate was prepared, a mixed powder was used, and alumina ball coating was performed such that the weight ratio of Cu and Si, the type of fiber-based binder, the amount of powder used, and the coating thickness satisfied the following Table 1.

**<Comparative Example 5>**

A powder was prepared in the same manner as in Example 1, except that in Example 1, only $Cu(NO_3)_2 \cdot 3H_2O$ was used and the fiber-based binder (silica wool) was not used. Thereafter, in order to coat a support to be described below with the powder, the powder, isopropyl alcohol (IPA), and water were mixed to prepare a mixed solution. In this case, it is named powder rather than mixed powder in a sense that no fiber-based binder was used.

Subsequently, an alumina ball with a size of 3.2 mm (strength of about 150 N) corresponding to the support was put into a chamber, and while spraying a mixed solution including only the $Cu(NO_3)_2 \cdot 3H_2O$, the alumina ball was coated with a mixed solution including only the $Cu(NO_3)_2 \cdot 3H_2O$. In this case, the coating thickness was 0.3 mm to 1 mm.

Thereafter, the alumina ball coated with the mixed solution including only the $Cu(NO_3)_2 \cdot 3H_2O$ was dried at 80°C for 7 hours using an oven, and the dried alumina ball coated with the mixed solution was fired in a firing furnace at a temperature of 250°C to 550°C for 8 hours to prepare the catalyst for preparing neopentyl glycol in Comparative Example 1.

**<Comparative Example 6>**

A catalyst for preparing neopentyl glycol in Comparative Example 6 was prepared in the same manner as in Example 1, except that hydroxypropyl methylcellulose (HPMC) was used instead of ceramic wool in Example 1. The hydroxypropyl methylcellulose (HPMC) is not a fiber-based binder, but a material corresponding to an organic binder.

The catalysts for preparing neopentyl glycol of Examples 1 to 9 and Comparative Examples 1 to 8 were analyzed by XRD to measure the content of CuO, and the results are shown in the following Table 1. In this case, in order to measure the content of CuO by performing XRD analysis, the diffraction angle (2θ) of CuK-alpha characteristic X-ray wavelength was measured through X-ray diffraction (XRD) analysis.

More specifically, as a spike for quantification during the measurement of the content of CuO, MgO heat-treated at 800°C for 5 hours was introduced at arbitrary contents (5, 10, and 20 wt%) to calculate the relative content of crystalline CuO and amorphous Cu-silicate.

In addition, the types of materials used in the catalysts for preparing neopentyl glycol in Examples 1 to 9 and Comparative Examples 1 to 8, the weight ratio of Cu and Si (Cu:Si), the coating thickness, and the amount of powder used are also shown in the following Table 1.

**<Reference Example 1>**

In order to evaluate the performance of the catalysts for preparing neopentyl glycol of Examples 1 to 9 and Comparative Examples 1 to 8, existing extruded catalysts (CuO/SiO$_2$ with a CuO content of 40 wt%) were prepared. The catalyst of Reference Example 1 was prepared in the same manner as in Example 1 up to the process of preparing a coprecipitate cake using a coprecipitate with a weight ratio of Cu and Si of Cu:Si=40:60 and a fiber-based binder, and was finally prepared in a size of 5 mm x 5 mm using an extruder without coating the support.

**<Reference Example 2>**

In order to evaluate the performance of the catalysts for preparing neopentyl glycol of Examples 1 to 9 and Comparative Examples 1 to 8, existing tableted catalysts (CuO/SiO$_2$ with a CuO content of 40 wt%) were prepared. The catalyst of Reference Example 2 was prepared in the same manner as in Example 1 up to the process of preparing a coprecipitate cake using a coprecipitate with a weight ratio of Cu and Si of Cu:Si=40:60 and a fiber-based binder, and was finally prepared in a size of 5 mm x 5 mm using a tablet press without coating the support.

Information on the catalysts for preparing neopentyl glycol in Reference Examples 1 and 2 is also shown in the following Table 1. For reference, the types of alumina balls are the same, so that the alumina balls are not shown in the following Table 1.

In order to obtain the weight ratio of Cu and Si (Cu:Si in the following Table 1), the content of Cu may be measured by Inductively Coupled Plasma Optical Emission Spectrometer (ICP-OES) analysis. The content of Cu was measured using an Optima 8300DV apparatus manufactured by PerkinElmer Inc. Specifically, the measured catalyst and nitric acid were put into a platinum crucible, and then diluted with ultrapure water to a concentration of 10 M. Thereafter, the content of Cu was calculated according to the following Mathematical Formula 1. Thereafter, the content of Si was obtained according to the following Mathematical Formula 2.

Content (wt%) of Cu = concentration to be measured (μg/mL) × (diluted volume (mL) / weight (μg) of catalyst) × 100 [Mathematical Formula 1]

[Mathematical Formula 2]

Content (wt%) of Si = 100 wt% - content (wt%) of Cu

[Table 1]

| No. | Type of catalyst (Type of fiber-based binder) | Cu:Si | CuO content (wt%) | Coating thickness (mm) | Amount of powder used (%) |
|---|---|---|---|---|---|
| Example 1 | CuO/SiO$_2$ (ceramic wool) | 40:60 | 20 | 2 ~ 4 | 100 |
| Example 2 | CuO/SiO$_2$ (ceramic wool) | 50:50 | 20 | 1 ~ 2 | 70 |
| Example 3 | CuO/SiO$_2$ (ceramic wool) | 50:50 | 10 | 0.3 ~ 1 | 26 |
| Example 4 | CuO/SiO$_2$ (ceramic wool) | 40:60 | 10 | 1 ~ 2 | 38 |
| Example 5 | CuO/SiO$_2$ (ceramic wool) | 50:50 | 13 | 2 ~ 4 | 51 |
| Example 6 | CuO/SiO$_2$ (carbon fiber) | 40:60 | 20 | 2 ~ 4 | 100 |
| Example 7 | CuO/SiO$_2$ (alumina fiber) | 40:60 | 20 | 2 ~ 4 | 100 |
| Example 8 | CuO/SiO$_2$ (ceramic wool) | 30:70 | 18 | 3 ~ 5 | 130 |
| Example 9 | CuO/SiO$_2$ (ceramic wool) | 70:30 | 22 | 1 ~ 2 | 58 |
| Comparative Example 1 | CuO/SiO$_2$ (ceramic wool) | 15:85 | 20 | 5 ~ 7 | 211 |
| Comparative Example 2 | CuO/SiO$_2$ (ceramic wool) | 40:60 | 1 | 2 ~ 4 | 100 |
| Comparative Example 3 | CuO/SiO$_2$ (ceramic wool) | 40:60 | 50 | 2 ~ 4 | 100 |
| Comparative Example 4 | CuO/SiO$_2$ (ceramic wool) | 80:20 | 20 | 0.7 ~ 1.5 | 42 |
| Comparative Example 5 | CuO | 100:0 | 10 | 0.3 ~ 1 | - |
| Comparative Example 6 | CuO/SiO$_2$ (HPMC) | 40:60 | 20 | 2 ~ 4 | 100 |
| Comparative Example 7 | CuO/SiO$_2$ (carbon fiber) | 15:85 | 20 | 5 ~ 7 | 211 |
| Comparative Example 8 | CuO/SiO$_2$ (alumina fiber) | 15:85 | 20 | 5 ~ 7 | 211 |
| Reference Example 1 | CuO/SiO$_2$, extruded catalyst | 40:60 | 40 | - | 142 |
| Reference Example 2 | CuO/SiO$_2$, tableted catalyst | 40:60 | 40 | - | 179 |

In Table 1 above, the material described in the parenthesis for the type of catalyst means the type of binder used, and Cu:Si means the weight ratio of Cu and Si in the coprecipitate or catalyst.

In this case, it was separately described that Reference Example 1 corresponds to an extruded catalyst and Reference Example 2 corresponds to a tableted catalyst.

Furthermore, the CuO content indicates the content of CuO in the catalyst, and for the coating thickness, as described above, the coating thickness means a calculated value obtained by dividing a value obtained by subtracting the size of the alumina ball from the size of the catalyst for preparing neopentyl glycol by 2, and the amount of powder used means the relative amount used (%) when the amount used (based on mass) of Example 1 is defined as 1 (100%).

In the case of Comparative Example 5, only a copper precursor was used, and Reference Examples 1 and 2 correspond to existing extruded catalyst and tableted catalyst, so that the amount of powder used was not described.

**<Experimental Example 1>**

Neopentyl glycol was prepared using a fixed bed reactor (FBR) filled with each of the catalysts for preparing neopentyl glycol of Reference Examples 1 and 2, Examples 1 to 9, and Comparative Examples 1 to 8. Specifically, a hydrogenation reaction was carried out in an air atmosphere under the conditions of a temperature of 160°C and a pressure of 35 bar for 1 hour using a reaction raw material consisting of 65 wt% hydroxypivaldehyde (HPA), 2 wt% neopentyl glycol (NPG), 25 wt% 2-isopropyl ethanol (2-EH), 5 wt% water (H$_2$O), and 3 wt% HPNE.

In this case, the amount of hydrogen consumed was measured to determine the catalyst activity of each of the catalysts for preparing neopentyl glycol of Reference Examples 1 and 2, Examples 1 to 9, and Comparative Examples 1 to 8.

The results are shown in the following Table 2.

**<Experimental Example 2>**

The Si content of each of the catalysts for preparing neopentyl glycol of Reference Examples 1 and 2, Examples 1 to 9, and Comparative Examples 1 to 8 was measured by strength and ICP-OES analysis.

For the stability test, the catalysts for preparing neopentyl glycol of Reference Examples 1 and 2, Examples 1 to 9, and Comparative Examples 1 to 8 were immersed in the liquid reaction raw material of Experimental Example 1 for reaction at a temperature of 180°C in a hydrogen atmosphere for 6 hours.

As another form of the stability test, the catalysts for preparing neopentyl glycol of Reference Examples 1 and 2, Examples 1 to 9, and Comparative Examples 1 to 8 were immersed in a neopentyl glycol solution for reaction at a temperature of 180°C in a hydrogen atmosphere for 18 hours.

Thereafter, the strength of the catalysts for preparing neopentyl glycol of Reference Examples 1 and 2, Examples 1 to 9, and Comparative Examples 1 to 8 after the reaction was measured and compared with the strength of the catalyst measured before the reaction to confirm the difference.

Thereafter, the Si content of the catalysts for preparing neopentyl glycol of Reference Examples 1 and 2, Examples 1 to 9, and Comparative Examples 1 to 8 after the stability test reaction under the reaction raw material conditions of Experimental Example 1 was measured by ICP-OES analysis and compared with the Si content of the catalyst measured before the reaction to confirm the difference.

The results are shown in the following Table 2.

[Table 2]

| No. | Physical properties | | | | Catalyst activity (%) |
|---|---|---|---|---|---|
| | Fresh catalyst | Strength (N) after stability test | | Si elution | |
| | strength (N) | Reaction raw material conditions | Product conditions | (%) | |
| Example 1 | 45 | 47 | 52 | 4 | 100 |
| Example 2 | 56 | 58 | 59 | 2 | 119 |
| Example 3 | 78 | 78 | 79 | 2 | 117 |
| Example 4 | 81 | 77 | 78 | 2 | 93 |
| Example 5 | 69 | 66 | 69 | 2 | 99 |
| Example 6 | 48 | 48 | 50 | 2 | 115 |
| Example 7 | 51 | 50 | 54 | 2 | 98 |
| Example 8 | 72 | 73 | 71 | 3 | 116 |
| Example 9 | 50 | 53 | 51 | 0 | 121 |
| Comparative Example 1 | 33 | 17 | - | 13 | 89 |
| Comparative Example 2 | 42 | 38 | - | 11 | 75 |
| Comparative Example 3 | 32 | 27 | - | 14 | 73 |
| Comparative Example 4 | 89 | 89 | - | 2 | 55 |
| Comparative Example 5 | 125 (based on crushing) | - | - | - | Reactivity None |
| Comparative Example 6 | 14 | - | - | - | 113 |
| Comparative Example 7 | 28 | 12 | - | 18 | 78 |
| Comparative Example 8 | 25 | 15 | - | 15 | 80 |

(continued)

| No. | Physical properties | | | | Catalyst activity (%) |
| | Fresh catalyst | Strength (N) after stability test | | Si elution | |
| | strength (N) | Reaction raw material conditions | Product conditions | (%) | |
| Reference Example 1 | 72 (based on crushing) | 51 (reduced by 29%) | 55 (reduced by 24%) | 27 | 84 |
| Reference Example 2 | 55 | 57 | 58 | 2 | 79 |

In Table 2 above, the Si elution (%) is a value calculated as [{Si content (weight) of initial catalyst (before reaction) - Si content (weight) of catalyst after stability test (after reaction) }/Si content (weight) of initial catalyst (before reaction)] × 100.

Further, the catalyst activity is a value indicating relative catalyst activity when the catalyst activity (unit: mL/min · Cat mL) of Example 1 is set to 1 as a reference (100%). That is, for example, when the catalytic activity of Example 1 is 100 mL/min · Cat mL, this means that the catalyst activity of Example 2 is 119 mL/min · Cat mL. In addition, the examples are arbitrary values and do not mean the activity values of the actual catalyst.

From the results in Table 2, it could be confirmed that the catalysts for preparing neopentyl glycol of Examples 1 to 9 have excellent catalyst activity as coating catalysts when compared to the extruded catalyst of Reference Example 1 and the tableted catalyst of Reference Example 2.

Furthermore, it could be confirmed that the catalysts for preparing neopentyl glycol of Examples 1 to 9 were used as coating catalysts, had fresh strengths similar to those of the extruded catalyst of Reference Example 1 and the tableted catalyst of Reference Example 2, and showed a small change in strength after the stability test and an amount of Si eluted. A small change in strength and a small amount of Si eluted mean that the catalyst has excellent physical stability. That is, this means that the durability of the catalyst is improved, which has an advantage of being able to improve the stability of the catalyst process and the service life of the catalyst.

Further, the catalysts for preparing neopentyl glycol in Examples 6 and 7 are coated catalysts using carbon fibers and alumina fibers as fiber-based binders, respectively, and were confirmed to have physical stability by confirming that the catalysts had fresh strength similar to that of the coated catalyst using ceramic wool as a fiber-based binder in Example 1 and had a low amount of Si eluted. That is, this means that as long as the binder is a usable fiber-based binder, the binder is not limited to ceramic wool, and exhibits the desired effect.

In addition, it could also be confirmed that Comparative Examples 1 to 3 and 5 to 8 were not physically stable compared to the Examples.

Specifically, Comparative Example 1 is a case where the weight of Cu in the coprecipitate is lower than the weight of Si as in the Examples, but the specific weight ratio of Cu and Si in the coprecipitate does not satisfy the weight ratio of the catalyst for preparing neopentyl glycol according to the present invention, and it could be confirmed that the coating thickness had to be extremely thick when copper with the same content as in Example 1 needs to be supported in order to secure the performance of the catalyst. When the coating thickness becomes extremely thick, the physical stability of the catalyst will deteriorate. That is, since the catalyst of Comparative Example 1 does not have good catalyst durability, there is a problem in that the stability of the catalyst process operation and the service life of the catalyst are reduced.

Comparative Examples 2 and 3 are cases where the weight ratio of Cu and Si of the catalyst for preparing neopentyl glycol according to the present invention is satisfied, but the content of CuO deviates from the content of the catalyst for preparing neopentyl glycol, and it could be confirmed that the catalyst activity was not good and even after the stability test, the change in strength and the amount of Si eluted were large.

Comparative Example 4 is a case where the weight of Cu in the coprecipitate is greater than the weight of Si, and even in this case, it could be confirmed that the catalyst activity was not good. It is determined that the activity is reduced because the size of the particles of Cu, which is known to be an active material in hydrogenation reactions, became larger than those in the Examples.

Comparative Example 5 is a catalyst for preparing neopentyl glycol prepared by coating with a material including only Cu, unlike the catalysts for preparing neopentyl glycol using a coprecipitate including Cu and Si in Examples 1 to 9. That is, Comparative Example 5 also corresponds to a coated catalyst.

However, when identically compared under the conditions in Experimental Example 1, it could be confirmed that Comparative Example 5 did not exhibit catalyst activity. This is determined to be due to the effect that the growth of the particle size of Cu, which is known as an active material in hydrogenation reactions, is regulated by Si for the catalysts for preparing neopentyl glycol (coated catalysts) in the Examples. That is, in Comparative Example 5, it is determined that the particle size of Cu became large enough to have no catalyst activity, as in Comparative Example 4.

Comparative Example 6 is a coated catalyst prepared using a coprecipitate including Cu and Si, like the catalysts for preparing neopentyl glycol in Examples 1 to 9, and was determined that the catalyst activity is similar to that in the Examples, but the coating strength is so weak that it is impossible to use the catalyst in the catalytic process. Comparative Example 6 is a case where a fiber-based binder was not used, and it could be confirmed that it is effective in increasing the strength (durability) of the catalyst to use a fiber-based binder as in the catalyst for preparing neopentyl glycol according to the present invention.

In addition, the catalysts for preparing neopentyl glycol in Comparative Examples 7 and 8 are coated catalysts using carbon fibers and alumina fibers as fiber-based binders, respectively, and are prepared in the same manner as the coated catalyst using ceramic wool in Comparative Example 1, and the fresh strength was low and Si elution occurred at a high rate in spite of the change in the fiber-based binder, as in Comparative Example 1. That is, this means that even though the type of fiber-based binder used is changed, the effect that the physical stability is improved by the fiber-based binder cannot be secured when the Cu:Si ratio of the coprecipitate deviates from the appropriate range.

That is, since the catalyst for preparing neopentyl glycol according to the present invention can maintain the activity of the catalyst even in a high-temperature and high-pressure reaction due to the excellent strength, reactivity and stability of the catalyst, it could be confirmed that the stability of the process for preparing neopentyl glycol can be enhanced and the activity of the catalyst is excellent.

As a result, when the catalyst for preparing neopentyl glycol according to the present invention is used, the process may be performed for a long time and the yield of neopentyl glycol may be increased, so that it could be confirmed that there is an advantage of being able to increase productivity.

## Claims

1. A catalyst for preparing neopentyl glycol (NPG), comprising:

   a support;
   a fiber-based binder; and
   CuO and $SiO_2$,
   wherein the weight ratio of Cu and Si is 30 : 70 to 70 : 30, and
   the catalyst for preparing neopentyl glycol comprises CuO in an amount of 5 parts by weight to 40 parts by weight based on 100 parts by weight of the catalyst by XRD analysis.

2. The catalyst of claim 1, wherein the catalyst comprises $CuO/SiO_2$, $CuO/BaO/SiO_2$, $CuO/ZnO/SiO_2$, $CuO/Al_2O_3/SiO_2$, $CuO/MnO/SiO_2$ or $CuO/Cr_2O_3/SiO_2$.

3. The catalyst of claim 1, wherein the fiber-based binder is one or more selected from the group consisting of glass fiber, carbon fiber, aramid fiber, alumina fiber, silicon carbide fiber, and boron fiber.

4. The catalyst of claim 1, wherein the support has a packing density of 600 $kg/m^3$ to 1500 $kg/m^3$.

5. The catalyst of claim 1, wherein the support comprises zirconium oxide, aluminum silicate oxide, silicon oxide or aluminum oxide.

6. A method for preparing a catalyst for preparing neopentyl glycol, the method comprising:

   preparing a coprecipitate comprising a copper (Cu) precursor and a silica ($SiO_2$) sol;
   drying the coprecipitate;
   preparing a coating powder by adding a fiber-based binder to the dried coprecipitate; and
   coating a support with the coating powder using an aqueous alcohol solution,
   wherein the catalyst for preparing neopentyl glycol is the catalyst of any one of claims 1 to 5.

7. The method of claim 6, wherein the fiber-based binder is comprised in an amount of 5 parts by weight to 20 parts by weight based on 100 parts by weight of the coprecipitate.

8. The method of claim 6, wherein the preparing of the coating powder by adding the fiber-based binder to the dried coprecipitate comprises:

pulverizing a material in which the fiber-based binder is added to the dried coprecipitate; and
preparing a powder by classifying the pulverized material.

9. The method of claim 8, wherein the classification range of the powder is 5 $\mu$m to 100 $\mu$m.

10. The method of claim 6, wherein the weight ratio of copper (Cu) and silicon (Si) in the coprecipitate is 20 : 80 to 50 : 50.

11. The method of claim 6, wherein the aqueous alcohol solution is an aqueous solution comprising one or more selected from the group consisting of methanol, ethanol, and isopropyl alcohol.

12. The method of claim 11, wherein the aqueous alcohol solution comprises the alcohol in an amount of 0.1 parts by weight to 80 parts by weight based on 100 parts by weight of the aqueous alcohol solution.

[Figure 1]

# INTERNATIONAL SEARCH REPORT

| | |
|---|---|
| International application No. | |
| | **PCT/KR2024/095165** |

## A. CLASSIFICATION OF SUBJECT MATTER

**B01J 23/72**(2006.01)i; **B01J 21/08**(2006.01)i; **B01J 35/51**(2024.01)i; **B01J 35/40**(2024.01)i; **B01J 37/03**(2006.01)i; **B01J 37/02**(2006.01)i; **C07C 29/141**(2006.01)i; **C07C 31/20**(2006.01)i

According to International Patent Classification (IPC) or to both national classification and IPC

## B. FIELDS SEARCHED

Minimum documentation searched (classification system followed by classification symbols)

B01J 23/72(2006.01); B01J 21/04(2006.01); B01J 23/88(2006.01); B01J 23/888(2006.01); B01J 27/199(2006.01); B01J 27/228(2006.01); B01J 35/02(2006.01); C07C 29/136(2006.01); C07C 29/141(2006.01); C07C 31/20(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 네오펜틸 글리콜(neopentyl glycol), 촉매(catalyst), 섬유계 바인더(fiber-based binder), 지지체(support), CuO, SiO2

## C. DOCUMENTS CONSIDERED TO BE RELEVANT

| Category* | Citation of document, with indication, where appropriate, of the relevant passages | Relevant to claim No. |
|---|---|---|
| Y | KR 10-2021-0037323 A (LG CHEM, LTD.) 06 April 2021 (2021-04-06)<br>See paragraphs [0012], [0034], [0036] and [0046]. | 1-12 |
| Y | JP 5628936 B2 (NIPPON SHOKUBAI CO., LTD.) 19 November 2014 (2014-11-19)<br>See paragraphs [0007], [0015] and [0018]; and example 1. | 1-12 |
| A | KR 10-2018-0129189 A (LG CHEM, LTD.) 05 December 2018 (2018-12-05)<br>See entire document. | 1-12 |
| A | CN 112537998 A (SHANGHAI E.TURN TECHNOLOGY CO., LTD. et al.) 23 March 2021 (2021-03-23)<br>See entire document. | 1-12 |
| A | KR 10-2020-0128734 A (MITSUBISHI CHEMICAL CORPORATION) 16 November 2020 (2020-11-16)<br>See entire document. | 1-12 |

☐ Further documents are listed in the continuation of Box C.    ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **29 May 2024** | **31 May 2024** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**
Information on patent family members

International application No.

**PCT/KR2024/095165**

| Patent document cited in search report | | | Publication date (day/month/year) | Patent family member(s) | | | Publication date (day/month/year) |
|---|---|---|---|---|---|---|---|
| KR | 10-2021-0037323 | A | 06 April 2021 | None | | | |
| JP | 5628936 | B2 | 19 November 2014 | CN | 103228356 | A | 31 July 2013 |
| | | | | CN | 103228356 | B | 23 September 2015 |
| | | | | EP | 2647429 | A1 | 09 October 2013 |
| | | | | EP | 2647429 | A4 | 13 August 2014 |
| | | | | EP | 2647429 | B1 | 06 September 2023 |
| | | | | JP | WO2012-073584 | A1 | 19 May 2014 |
| | | | | US | 2013-0253223 | A1 | 26 September 2013 |
| | | | | US | 8940658 | B2 | 27 January 2015 |
| | | | | WO | 2012-073584 | A1 | 07 June 2012 |
| KR | 10-2018-0129189 | A | 05 December 2018 | KR | 10-2297983 | B1 | 02 September 2021 |
| CN | 112537998 | A | 23 March 2021 | CN | 112537998 | B | 23 December 2022 |
| KR | 10-2020-0128734 | A | 16 November 2020 | CN | 111727085 | A | 29 September 2020 |
| | | | | JP | 6973618 | B2 | 01 December 2021 |
| | | | | JP | WO2019-177031 | A1 | 19 September 2019 |
| | | | | KR | 10-2547450 | B1 | 23 June 2023 |
| | | | | WO | 2019-177031 | A1 | 19 September 2019 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020230028645
- KR 1020240020773
- KR 1020110038324